# EUROPÄISCHE PATENTSCHRIFT

(11) **EP 1 685 383 B1**
(45) Veröffentlichungstag und Bekanntmachung des Hinweises auf die Patenterteilung: **11.02.2015**
(21) Anmeldenummer: 04789968.7
(22) Anmeldetag: 12.10.2004
(51) Int. Cl.: G01N 15/08

(54) **VERFAHREN ZUR BESTIMMUNG DER GASDURCHLÄSSIGKEIT VON BEHÄLTERWANDUNGEN**
METHOD FOR DETERMINING THE GAS PERMEABILITY OF CONTAINER WALLS
PROCEDE POUR DETERMINER LA PERMEABILITE AUX GAZ DE PAROIS DE RECIPIENTS

(30) Priorität: 22.11.2003 DE 10354625
(43) Veröffentlichungstag der Anmeldung: 02.08.2006
(73) Patentinhaber: KHS Corpoplast GmbH, 22145 Hamburg (DE)
(72) Erfinder: LÜPKE, Erik, 22089 Hamburg (DE); HARTWIG, Klaus, 54000 Nancy (FR)
(74) Vertreter: Hausfeld, Norbert
(86) Internationale Anmeldenummer: PCT/DE2004/002263
(87) Internationale Veröffentlichungsnummer: WO 2005/052555

(56) Entgegenhaltungen:
- WO-A-01/48452
- WO-A-02/059557
- DE-A1- 19 946 080
- US-A- 4 391 128
- US-A- 6 009 743
- US-A1- 2002 173 040

## Beschreibung

Die Erfindung betrifft ein Verfahren zur Bestimmung der Gasdurchlässigkeit von Wandungen eines Behälters, der mindestens im Bereich einer ersten Oberfläche der Wandungen mit einer Barrierebeschichtung versehen ist, sowie bei dem in einem mindestens bereichsweise von der Barrierebeschichtung begrenzten Analyseraum ein Gehalt an einem Analysegas gemessen wird, das aus dem Bereich der Wandung des Behälters in den Analyseraum austritt und vor seinem Austritt in den Analyseraum im Bereich der Wandung gebunden war.

Viele der bekannten Verfahren und Vorrichtungen zur Bestimmung der Gasdurchlässigkeit von Wandungen eines Behälters, insbesondere zur Bestimmung der Gasdurchlässigkeit von Behältern aus thermoplastischen Kunststoffen, weisen den Nachteil auf, daß ein erheblicher Zeitraum für die Durchführung der Messungen erforderlich ist. Im Zusammenhang mit der Messung einer Gasdurchlässigkeit von Wandungen von Flaschen aus PET sind beispielsweise in der deutschen Norm DIN 53380 beschriebene Verfahren bekannt, die eine typische Meßdauer von 5 bis 7 Tagen erfordern. Derartige Verfahren sind somit zwar geeignet, grundsätzliche Aussagen hinsichtlich der Anwendbarkeit bestimmter Verfahren oder der Materialeigenschaften bestimmter Wandungskonstruktionen zu treffen, für einen kontinuierlichen Einsatz zur Produktionsüberwachung mit frühzeitiger Erkennung von Produktionsstörungen sind die bekannten Verfahren und Vorrichtungen aber nicht geeignet. Bekannt sind auch Meßverfahren unter Verwendung von Wasserstoffperoxid, diese sind aber ungenau und bedienerunfreundlich.

Ein vergleichsweise schnell durchzuführendes Verfahren zur Messung der Barriereeigenschaften wird in der DE 101 24 225 A1 beschrieben. Nachteilig ist jedoch, daß zur meßtechnischen Durchführung ein Unterdruck innerhalb der Behälter generiert werden muß. Dies führt zu einem erhöhten apparativen Aufwand und erschwert insbesondere die Integration des Meßverfahrens in eine Produktionsüberwachung.

Zur Verringerung der Gasdurchlässigkeit von Wandungen von Behältern aus thermoplastischen Materialien sind grundsätzlich bereits eine Vielzahl von Verfahren bekannt. Eine weite Verbreitung hat inzwischen ein mehrlagiger Aufbau der Wandungen gefunden, bei dem typischerweise eine mittlere Wandungsschicht eine hohe Gassperrwirkung aufweist. Bekannt ist es ebenfalls, bestimmte Copolymere zu verwenden, die aufgrund ihrer molekularen Struktur ein relativ dichtes Gefüge bereitstellen, das einen Gasdurchtritt verhindert. Ebenfalls ist es bekannt, sogenannte Opfersubstanzen in das Material der Wandungen einzubetten, die eindringende Gaspartikel anlagern und hierdurch einen Durchtritt verhindern.

Gemäß weiteren bekannten Verfahren wird eine erhöhte Gasdichtigkeit bei einer blastechnischen Herstellung von thermoplastischen Behältern durch spezielle Reckbedingungen, Temperaturkonditionierungen sowie Blasdruckverläufe erreicht. Schließlich ist es ebenfalls bereits bekannt, die Behälterwandungen auf der inneren und/oder äußeren Oberfläche mit speziellen Beschichtungen zu versehen. In jüngster Zeit werden derartige Beschichtungen auch unter Verwendung von Plasmatechnologien erzeugt.

Unabhängig von der konkreten Realisierung einer erhöhten Gasundurchlässigkeit der jeweiligen Behälterwandungen können die bislang bekannten Verfahren und Vorrichtungen zur Bestimmung der Gasundurchlässigkeit noch nicht alle Anforderungen an eine schnelle Meßdurchführung, eine hohe Meßgenauigkeit sowie eine ausreichende Störungsunempfindlichkeit erfüllen.

Aus der US 4,391,128 A ist es bereits bekannt, einen Behälter im Bereich seiner Oberfläche mit einer Barrierebeschichtung zu versehen. Zur Analyse der Qualität der Barriereschicht wird in einem Analyseverfahren eine Analysesubstanz durch die Barriereschicht hindurch in die Wandung des Behältermaterials eindiffundiert und anschließend die Rückdiffusion messtechnisch erfasst.

In der WO 02/059557 A wird ein Verfahren beschrieben, um die Dichtigkeit von versiegelten Blisterverpackungen zu überprüften. In die Verpackung wird hierzu ein Analysegas eingebracht und es wird anschließend untersucht, ob das Analysegas gegebenenfalls durch Fehlstellen der Siegelnaht entweicht.

Die WO 01/004845 A beschreibt ein Verfahren zur Ermittlung der Dichtigkeit von Barriereschichten von Flaschen. Es wird hierzu ein Analysegas in den Innenraum der Flasche eingebracht und anschließend die Diffusion durch die Barriereschicht und durch die Wandung der Flasche hindurch in einen die Flasche umgebenden Auswertungsraum gemessen.

In der DE 199 46 080 A1 wird eine Testflasche beschrieben, die mit einer Kohlenstoffschicht versehen ist. Die Beschichtung dient zu einer Aufbringung von Markierungen, die optisch erfasst werden können.

Aufgabe der vorliegenden Erfindung ist es, ein Verfahren der einleitend genannten Art derart zu verbessern, daß eine Erhöhung der Analysegeschwindigkeit bei gleichzeitig hoher Analysequalität erreicht wird.

Diese Aufgabe wird erfindungsgemäß dadurch gelöst, daß im Bereich der der Barrierebeschichtung zugewandten Oberfläche der Wandung mindestens eine Analysesubstanz angeordnet ist, die mindestens zeitweilig ein Analysegas abgibt, und dass das Analysegas innerhalb eines Innenraumes des Behälters oder in einem dem Behälter umgebendem Bezugsraum gemessen wird.

Durch die Verwendung einer Substanz als Analysegas, die im Bereich der Behälterwandung derart lose gebunden ist, daß ein Austritt aus der Behälterwandung möglich ist, wird unmittelbar unterhalb der Barrierebeschichtung bereits sofort nach einem Abschluß des Beschichtungsvorganges eine für die Analyse nutzbare Substanz bereitgestellt. Es ist somit nicht erforderlich, den beschichteten Behälter zunächst nach der Durchführung der Beschichtung einer analysegashaltigen Umgebung auszusetzen und eine Sättigung des Materials des Behälters mit dem Analysegas abzuwarten. Diese typischerweise 30 bis 60 Minuten betragende Sättigungszeit kann durch die unmittelbare Präsenz der Analysesubstanz im Bereich der Behälterwandung vermieden werden. Es lassen sich hierdurch Analysezeiten im Bereich weniger Minuten realisieren. Unter der Behälterwandung, deren Gasdurchlässigkeit zu ermitteln ist, wird im folgenden die Kombination der eigentlichen Wandung und der Barrierebeschichtung verstanden.

Bei Behältern mit einer Innenbeschichtung erweist es sich erfindungsgemäß als zweckmäßig, daß das Analysegas innerhalb eines Innenraumes des Behälters gemessen wird.

Bei Behältern mit einer Außenbeschichtung ist es erfindungsgemäß vorteilhaft, daß das Analysegas in einem dem Behälter umgebendem Bezugsraum gemessen wird.

Eine sehr hohe Meßqualität wird insbesondere dadurch unterstützt, daß Anteile des Analysegases in einem Raum gemessen werden, der vor Beginn der Messung praktisch frei von Analysegas ist. Hierdurch kann auch bei relativ geringen Mengen an zu detektierender Substanz eine sehr hohe Meßempfindlichkeit realisiert werden.

Aufgrund des gewählten Meßprinzips kann die Vorrichtung sehr kompakt konstruiert werden. Ebenfalls kann eine sehr geringe Störungsempfindlichkeit sowohl des Meßverfahrens als auch der Meßvorrichtung erreicht werden, so daß bei einer Integration der Meßvorrichtung in eine Vorrichtung zur Beschichtung von Behältern dauerhaft eine hohe Produktionsqualität unterstützt wird.

Eine technisch einfach zu realisierende Verfahrensvariante besteht darin, daß das Analysegas physikalisch im Bereich der Wandung gebunden wird.

Ebenfalls ist daran gedacht, daß das Analysegas chemisch im Bereich der Wandung gebunden wird.

Eine größere Menge an Analysesubstanz läßt sich dadurch bereitstellen, daß das Analysegas innerhalb der Wandung gebunden wird.

Sehr kurze Meßzeiten werden insbesondere dadurch unterstützt, daß das Analysegas in einer Zwischenschicht zwischen der Wandung und der Barrierebeschichtung gebunden wird.

Eine Verminderung der Analysesubstanz durch Lagerung des Behälters vor seiner Beschichtung wird dadurch vermieden, daß das Analysegas in einem der Wandung zugewandten Bereich der Barrierebeschichtung gebunden wird.

Eine einfache meßtechnische Verfahrensdurchführung wird insbesondere dadurch unterstützt, daß eine meßtechnische Erfassung des Analysegases bei einem Druck durchgeführt wird, der im wesentlichen gleich einem Umgebungsdruck gewählt wird.

Eine einfache Verfahrensdurchführung wird auch dadurch unterstützt, daß als Analysegas Wasserdampf verwendet wird.

Darüber hinaus ist daran gedacht, daß als Analysegas Sauerstoff verwendet wird.

Gemäß einer weiteren Ausführungsform ist es auch möglich, daß als Analysegas Stickstoff verwendet wird.

Ebenfalls kann eine Verfahrensdurchführung derart erfolgen, daß als Analysegas Kohlendioxyd verwendet wird.

Eine flexible Durchführung des Meßverfahrens kann dadurch erreicht werden, daß eine meßtechnische Erfassung des Analysegases innerhalb eines Trägergases durchgeführt wird.

Eine Barrieremessung nach einer Befüllung der Behälter kann dadurch erfolgen, daß eine meßtechnische Erfassung des Analysegases innerhalb einer Trägerflüssigkeit durchgeführt wird.

Zur Unterstützung einer hohen Meßgenauigkeit wird vorgeschlagen, daß der Meßraum vor einer Durchführung der Messung derart vorkonditioniert wird, daß er im wesentlichen frei von Analysegas ist.

Eine Integration der Prüfeinrichtung in die Beschichtungseinrichtung wird dadurch unterstützt, daß die Prüfeinrichtung mit einer Steuereinrichtung für die Beschichtungseinrichtung gekoppelt ist.

Eine typische Ausführungsform besteht darin, daß die Beschichtungseinrichtung zur Innenbeschichtung der Behälter ausgebildet ist.

Ebenfalls ist daran gedacht, daß die Beschichtungseinrichtung zur Außenbeschichtung der Behälter ausgebildet ist.

Gemäß einer weiteren Ausführungsform ist es auch möglich, daß die Beschichtungseinrichtung zur Plasmabeschichtung der Behälter ausgebildet ist.

In den Zeichnungen sind Ausführungsbeispiele der Erfindung schematisch dargestellt. Es zeigen:
- Fig. 1: eine schematische Darstellung der Meßeinrichtung,
- Fig. 2: ein Diagramm zur Veranschaulichung von Meßergebnissen und
- Fig. 3: ein Blockschaltbild zur Veranschaulichung einer Integration der Meßvorrichtung in eine Vorrichtung zur Beschichtung von Behältern

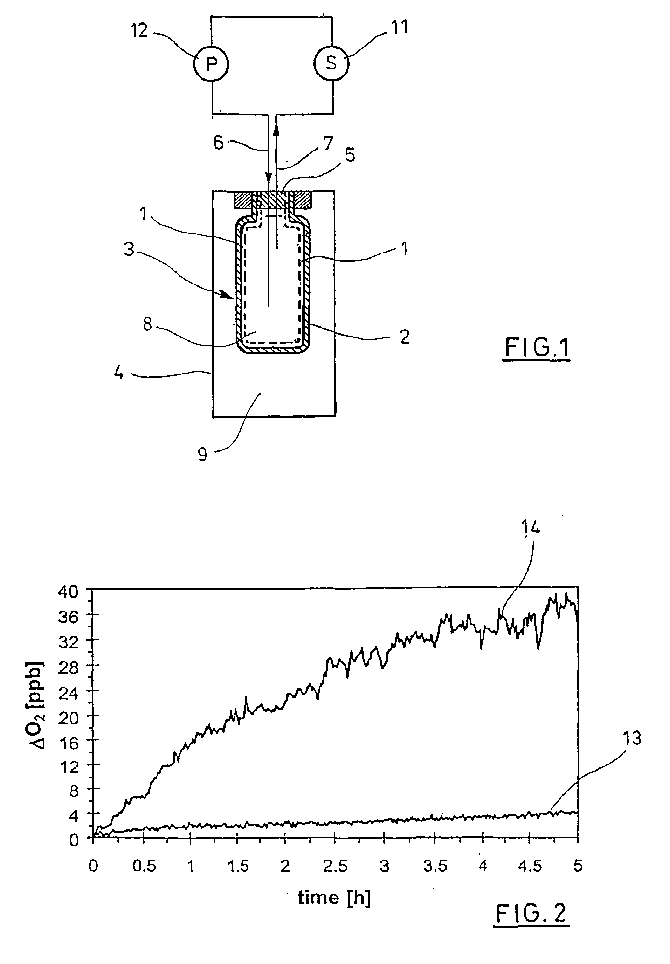

Fig. 1 zeigt eine Vorrichtung zur Bestimmung der Gasdurchlässigkeit einer Barrierebeschichtung (1) von Wandungen (2) eines Behälters (3). Der Behälter (3) ist in eine Halteeinrichtung (4) eingesetzt, die den Behälter (3) mindestens bereichsweise umschließt. Die Halteeinrichtung (4) besitzt einen Mulidungsverschluß (5) für den Behälter (3) mit einem Einlaß (6) sowie einem Auslaß (7) für ein Trägergas. Beim dargestellten Ausführungsbeispiel wird das Trägergas in einen Innenraum (8) des Behälters (3) eingeleitet. Das Trägergas kommt hierbei in Kontakt mit der Barrierebeschichtung (1) des Behälters (3), die beim dargestellten Ausführungsbeispiel dem Innenraum (8) zugewandt angeordnet ist.

Das Trägergas wird durch den Einlaß (6) von einer Pumpe (10) in den Innenraum (8) hineingeleitet und strömt über den Auslaß (7) an einem Sensor (11) vorbei.

Beim dargestellten Ausführungsbeispiel ist zur Einsparung von Trägergas ein geschlossener Gasumlauf vorgesehen. Grundsätzlich kann aber auch ein offenes System verwendet werden. Vor der Durchführung des Meßvorganges erfolgt typischerweise eine Spülung des Innenraumes (8) mit Trägergas, um definierte Anfangsbedingungen bereitzustellen.

Bei einer Ausführungsform, bei der die Barrierebeschichtung (1) außenseitig auf dem Behälter (3) angeordnet ist, wird die Halteeinrichtung (6) als geschlossene Kammer mit einem Bezugsraum (9) ausgebildet, in den der Behälter (3) eingesetzt wird. Der Einlaß (6) und der Auslaß (7) münden hierbei in den Bezugsraum (9) ein, damit das Trägergas die außenseitig aufgebrachte Barrierebeschichtung (1) umströmt. Ansonsten wird aber ein vergleichbares Meßprinzip realisiert.

Sowohl bei einer Anordnung der Barrierebeschichtung (1) auf einer inneren als auch auf einer äußeren Oberfläche der Wandung (2) erfaßt der Sensor (11) Anteile eines Analysegases am Trägergas, wobei das Analysegas durch die Barrierebeschichtung (1) hindurchtritt bzw. im Bereich unbeschichteter Flächen der Wandung (2) aus der Wandung (2) austritt. Die das Analysegas abgebende oder das Analysegas ausbildende Substanz kann bereits bei einer Produktion der Behälter (3) in die Wandung (2) eingebracht sein. Bei der blastechnischen Formung der Behälter (3) ist es zum einen möglich, bereits bei einem vorhergehenden spritzgußtechnischen Vorgang von Vorformlingen, die später in die Behälter (3) umgeformt werden, die betreffende Substanz in das Material einzubringen. Ebenfalls ist es möglich, die Substanz während oder im Anschluß an eine Blasverformung der Vorformlinge in die Behälter (3) auf einer Oberfläche der Wandung (2) abzulagern. Schließlich ist es auch möglich, die betreffende Substanz kurz vor einer Aufbringung der Barrierebeschichtung (1) auf der Wandung (2) abzulagern. Dies kann entweder in einem separaten Verfahrensschritt oder in einem ersten Beschichtungsschritt bei der Erzeugung einer mehrlagigen Beschichtung oder einer Gradientenbeschichtung erfolgen.

Insbesondere ist verfahrenstechnisch daran gedacht, die meßtechnische Erfassung des Analysegases bei Umgebungsdruck durchzuführen, ohne einen vorherigen Unterdruck im Meßraum zu erzeugen. Bei einem zuvor von Analysegas freien Meßraum tritt das Analysegas aufgrund von stoffspezifischen Partialdruckdifferenzen auch bei einem einwirkenden Umgebungsdruck aus dem Bereich der Wandung (2) aus.

Als Analysegas sind eine Vielzahl von Substanzen geeignet. Bei der Barrieremessung im Bereich von Behältern (3), die für die Verpackung von Lebensmitteln vorgesehen sind, ist insbesondere an die Verwendung von lebensmittelrechtlich zugelassenen Substanzen gedacht. Beispielsweise ist es möglich, im Bereich des Meßraumes zunächst ein getrocknetes Gas, beispielsweise trockene Luft, anzuordnen und einen Austritt von Wassermolekülen aus dem Bereich der Wandung (2) zu erfassen. Ebenfalls ist es möglich, einen Austritt von Sauerstoff oder Kohlendioxyd aus dem Bereich der Wandung meßtechnisch zu erfassen.

Alternativ zur Messung des Analysegases in einem Trägergas ist es auch möglich, die Messung in einer Trägerflüssigkeit durchzuführen. Bei einer Ausbildung der Behälter (3) als zu befüllende Flaschen ist insbesondere daran gedacht, als Trägerflüssigkeit eine hinsichtlich des Analysegases vorab entgaste Flüssigkeit zu verwenden und die Messung nach einer Befüllung des Behälters (3) durchzuführen.

Bei der Verwendung von Behältern (3), die zur Verpackung von Lebensmitteln vorgesehen sind, ist insbesondere daran gedacht, die Wandung (2) aus PET oder PEN zu realisieren und als Analysesubstanz Materialien zu verwenden, die in besonderer Weise für eine Anlagerung an PET oder PEN mit einer hinsichtlich der erforderlichen Substanzabgabe optimalen Anlagestabilität geeignet sind.

Hinsichtlich der Analysesubstanz ist insbesondere auch daran gedacht, Substanzen zu verwenden, die die Transparenz von transparenten Wandungen (2) nicht ungünstig beeinflussen und die nicht zu ungewollten Verfärbungen der Wandung (2) führen.

Fig. 2 zeigt hinsichtlich des Austrittes von Sauerstoff aus dem Bereich der Wandung (2) einen Verlauf (13) für einen Behälter (2) mit einer gleichmäßigen und wirkungsvollen Barrierebeschichtung (1) sowie einen Verlauf (14) für einen Behälter (3) ohne Barrierebeschichtung (1). Es ist zu erkennen, daß bereits nach wenigen Minuten die Verläufe (13, 14) signifikant voneinander abweichen.

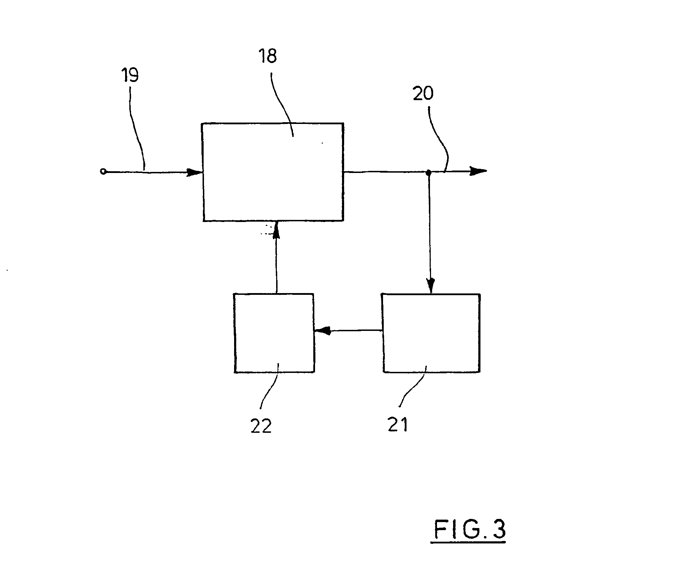

Fig. 3 zeigt eine Beschichtungseinrichtung (18) für die Behälter (3), die mit einer Behältereingabe (19) und einer Behälterausgabe (20) versehen ist. An die Behälterausgabe (20) ist eine Prüfeinrichtung (21) angeschlossen, die die Halteeinrichtung (4) mit Sensor (11) umfaßt. Die Prüfeinrichtung (21) ist an eine Steuereinrichtung (22) für die Beschichtungseinrichtung (18) angeschlossen, um in Abhängigkeit von den jeweiligen Meßergebnissen eine Anpassung von Maschineneinstellungen und/oder eine Funktionsanzeige vorzunehmen. Bei Beschichtungseinrichtungen mit hohen Produktionszahlen je Zeiteinheit können jeweils stichprobenartig beschichtete Behälter über eine gesteuerte Zuführung zur Prüfeinrichtung (21) transportiert werden. Eine Adaption der Steuerparameter der Steuereinrichtung (22) kann automatisch oder nach Benutzerfreigabe erfolgen.

Die Beschichtungseinrichtung kann zur Plasmabeschichtung der Behälter (3) ausgebildet sein. Gedacht ist insbesondere an eine Innenbeschichtung. Als Beschichtungsmaterial kann SIO_{X} verwendet werden, ggf. unter Einsatz eines Haftvermittlers.

## Patentansprüche

1. Verfahren zur Bestimmung der Gasdurchlässigkeit von Wandungen (2) eines Behälters (3), der mindestens im Bereich einer ersten Oberfläche der Wandungen (2) mit einer Barrierebeschichtung (1) versehen ist, sowie bei dem in einem mindestens bereichswelse von der Barrierebeschichtung (1) begrenzten Analyseraum ein Gehalt an einem Analysegas gemessen wird, das aus dem Bereich der Wandung (2) des Behälters (3) in den Analyseraum austritt und vor seinem Austritt in den Analyseraum im Bereich der Wandung (2) gebunden war, **dadurch gekennzeichnet, dass** im Bereich der der Barrierebeschichtung (1) zugewandten Oberfläche der Wandung (2) mindestens eine Analysesubstanz angeordnet ist, die mindestens zeitweilig ein Analysegas abgibt und dass das Analysegas innerhalb eines Innenraumes (8) des Behälters (3) oder in einem dem Behälter (3) umgebendem Bezugsraum (9) gemessen wird.

2. Verfahren nach Anspruch 1, **dadurch gekennzeichnet, dass** das Analysegas physikalisch im Bereich der Wandung (2) gebunden wird.

3. Verfahren nach Anspruch 1, **dadurch gekennzeichnet, dass** das Analysegas chemisch im Bereich der Wandung (2) gebunden wird.

4. Verfahren nach einem der Ansprüche 1 bis 3, **dadurch gekennzeichnet, dass** das Analysegas innerhalb der Wandung (2) gebunden wird.

5. Verfahren nach einem der Ansprüche 1 bis 3, **dadurch gekennzeichnet, dass** das Analysegas in einer Zwischenschicht zwischen der Wandung (2) und der Barrierebeschichtung (1) gebunden wird.

6. Verfahren nach einem der Ansprüche 1 bis 3, **dadurch gekennzeichnet, dass** das Analysegas in einem der Wandung (2) zugewandten Bereich der Barrierebeschichtung (1) gebunden wird.

7. Verfahren nach einem der Ansprüche 1 bis 6, **dadurch gekennzeichnet, dass** eine messtechnische Erfassung des Analysegases bei einem Druck durchgeführt wird, der im wesentlichen gleich einem Umgebungsdruck gewählt wird.

8. Verfahren nach einem der Ansprüche 1 bis 7, **dadurch gekennzeichnet, dass** als Analysegas Wasserdampf verwendet wird.

9. Verfahren nach einem der Ansprüche 1 bis 7, **dadurch gekennzeichnet, dass** als Analysegas Sauerstoff verwendet wird.

10. Verfahren nach einem der Ansprüche 1 bis 7, **dadurch gekennzeichnet, dass** als Analysegas Stickstoff verwendet wird.

11. Verfahren nach einem der Ansprüche 1 bis 7, **dadurch gekennzeichnet, dass** als Analysegas Kohlendioxyd verwendet wird.

12. Verfahren nach einem der Ansprüche 1 bis 11, **dadurch gekennzeichnet, dass** eine messtechnische Erfassung des Analysegases innerhalb eines Trägergases durchgeführt wird.

13. Verfahren nach einem der Ansprüche 1 bis 11, **dadurch gekennzeichnet, dass** eine messtechnische Erfassung des Analysegases innerhalb einer Trägerflüssigkeit durchgeführt wird.

14. Verfahren nach einem der Ansprüche 1 bis 13, **dadurch gekennzeichnet, dass** der Messraum vor einer Durchführung der Messung derart vorkonditioniert wird, dass er im wesentlichen frei von Analysegas ist.

15. Verfahren nach einem der Ansprüche 1 bis 14, **dadurch gekennzeichnet, dass** die Analysesubstanz gasförmig in der Wandung (2) gebunden wird.

16. Verfahren nach einem der Ansprüche 1 bis 15, **dadurch gekennzeichnet, dass** die Analysesubstanz flüssig in der Wandung (2) gebunden wird.

17. Verfahren nach einem der Ansprüche 1 bis 16, **dadurch gekennzeichnet, dass** eine die Analysesubstanz abgebende Trägersubstanz in der Wandung (2) gebunden wird.

18. Verfahren nach einem der Ansprüche 1 bis 17, **dadurch gekennzeichnet, dass** die Analysesubstanz in einem Rohstoff für die Herstellung der Behälter (2) gebunden wird.

19. Verfahren nach einem der Ansprüche 1 bis 18, **dadurch gekennzeichnet, dass** die Analysesubstanz in einem Vorprodukt für die Herstellung der Behälter (2) gebunden wird.

## Claims

1. A method for the determination of the gas permeability of walls (2) of a container (3) which is provided with a barrier coating (1) at least in the area of a first surface of the walls (2), and wherein a content of an analysis gas is measured in an analysis chamber that is delimited by the barrier coating (1) at least in certain regions, said analysis gas exiting from the area of the wall (2) of the container (3) and flowing into the analysis chamber and having been bound in the area of the wall (2) before it exited into the analysis chamber,
**characterised in that**
- at least one analysis substance is arranged in the area of the surface of the wall (2) that is facing the barrier coating (1), said analysis substance releasing an analysis gas at least temporarily, and that the analysis gas is measured inside an interior chamber (8) of the container (3) or in a reference chamber (9) surrounding the container (3).

2. The method according to Claim 1, **characterised in that** the analysis gas is bound physically in the area of the wall (2).

3. The method according to Claim 1, **characterised in that** the analysis gas is bound chemically in the area of the wall (2).

4. The method according to any one of Claims 1 to 3, **characterised in that** the analysis gas is bound inside the wall (2).

5. The method according to any one of Claims 1 to 3, **characterised in that** the analysis gas is bound in an intermediate layer between the wall (2) and the barrier coating (1).

6. The method according to any one of Claims 1 to 3, **characterised in that** the analysis gas is bound in an area of the barrier coating (1) facing the wall (2).

7. The method according to any one of Claims 1 to 6, **characterised in that** a metrological recording of the analysis gas is carried out at a pressure which is selected such that it is essentially equal to an ambient pressure.

8. The method according to any one of Claims 1 to 7, **characterised in that** water vapour is used as analysis gas.

9. The method according to any one of Claims 1 to 7, **characterised in that** oxygen is used as analysis gas.

10. The method according to any one of Claims 1 to 7, **characterised in that** nitrogen is used as analysis gas.

11. The method according to any one of Claims 1 to 7, **characterised in that** carbon dioxide is used as analysis gas.

12. The method according to any one of Claims 1 to 11, **characterised in that** a metrological recording of the analysis gas is carried out within a carrier gas.

13. The method according to any one of Claims 1 to 11, **characterised in that** a metrological recording of the analysis gas is carried out within a carrier liquid.

14. The method according to any one of Claims 1 to 13, **characterised in that** the measuring room is preconditioned prior to taking the measurement such that it is essentially free from analysis gas.

15. The method according to any one of Claims 1 to 14, **characterised in that** the analysis substance is bound in a gaseous form in the wall (2).

16. The method according to any one of Claims 1 to 15, **characterised in that** the analysis substance is bound in liquid form in the wall (2).

17. The method according to any one of Claims 1 to 16, **characterised in that** a carrier substance that releases the analysis substance is bound in the wall (2).

18. The method according to any one of Claims 1 to 17, **characterised in that** the analysis substance is bound in a raw material for the production of the containers (2).

19. The method according to any one of Claims 1 to 18, **characterised in that** the analysis substance is bound in an intermediate product for the production of the containers (2).

## Revendications

1. Procédé pour déterminer la perméabilité au gaz de parois (2) d'un récipient (3) doté d'un revêtement formant barrière (1) sur l'une au moins des surfaces des parois (2), dans le cadre duquel une teneur en gaz d'analyse est mesurée dans un espace d'analyse en partie au moins délimité par le revêtement formant barrière (1), ce gaz émanant de la zone de paroi (2) du récipient (3) dans l'espace d'analyse et ayant été lié dans cette zone de paroi (2) avant d'émaner dans l'espace d'analyse, **caractérisé en ce que** dans la zone de la surface de la paroi (2) se trouvant du côté du revêtement formant barrière (1) est prévue au moins une substance d'analyse qui, temporairement au moins, dégage un gaz d'analyse, lequel est mesuré à l'intérieur d'un espace intérieur (8) du récipient (3) ou dans un espace de référence (9) entourant le récipient (3).

2. Procédé selon la revendication 1, **caractérisé en ce que** le gaz d'analyse est lié physiquement dans la zone de paroi (2).

3. Procédé selon la revendication 1, **caractérisé en ce que** le gaz d'analyse est lié chimiquement dans la zone de paroi (2).

4. Procédé selon l'une des revendications 1 à 3, **caractérisé en ce que** le gaz d'analyse est lié à l'intérieur de la paroi (2).

5. Procédé selon l'une des revendications 1 à 3, **caractérisé en ce que** le gaz d'analyse est lié dans une couche intermédiaire entre la paroi (2) et le revêtement formant barrière (1).

6. Procédé selon l'une des revendications 1 à 3, **caractérisé en ce que** le gaz d'analyse est lié dans une zone du revêtement formant barrière (1) face à la paroi (2).

7. Procédé selon l'une des revendications 1 à 6, **caractérisé en ce qu**'une détermination métrologique du gaz d'analyse est effectuée sous une pression essentiellement égale à une pression ambiante.

8. Procédé selon l'une des revendications 1 à 7, **caractérisé en ce que** l'on utilise de la vapeur d'eau en guise de gaz d'analyse.

9. Procédé selon l'une des revendications 1 à 7, **caractérisé en ce que** l'on utilise de l'oxygène en guise de gaz d'analyse.

10. Procédé selon l'une des revendications 1 à 7, **caractérisé en ce que** l'on utilise de l'azote en guise de gaz d'analyse.

11. Procédé selon l'une des revendications 1 à 7r, **caractérisé en ce que** l'on utilise du gaz carbonique en guise de gaz d'analyse.

12. Procédé selon l'une des revendications 1 à 11, **caractérisé en ce que** l'on effectue une détermination métrologique du gaz d'analyse dans un gaz vecteur.

13. Procédé selon l'une des revendications 1 à 11, **caractérisé en ce que** l'on effectue une détermination métrologique du gaz d'analyse dans un liquide vecteur.

14. Procédé selon l'une des revendications 1 à 13, **caractérisé en ce que**, avant de procéder à la mesure, l'espace de mesure est préconditionné de façon à ce qu'il soit essentiellement exempt de gaz d'analyse.

15. Procédé selon l'une des revendications 1 à 14, **caractérisé en ce que** la substance d'analyse est liée sous forme gazeuse dans la paroi (2).

16. Procédé selon l'une des revendications 1 à 15, **caractérisé en ce que** la substance d'analyse est liée sous forme liquide dans la paroi (2).

17. Procédé selon l'une des revendications 1 à 16, **caractérisé en ce que** la substance d'analyse est dégagée par une substance porteuse liée dans la paroi (2).

18. Procédé selon l'une des revendications 1 à 17, **caractérisé en ce que** la substance d'analyse est liée dans une matière première utilisée pour la fabrication des récipients (2).

19. Procédé selon l'une des revendications 1 à 18, **caractérisé en ce que** la substance d'analyse est liée dans un produit semi-fini utilisé pour la fabrication des récipients (2).
